# EUROPÄISCHE PATENTSCHRIFT

(11) **EP 0 975 774 B1**
(45) Veröffentlichungstag und Bekanntmachung des Hinweises auf die Patenterteilung: **22.09.2004**
(21) Anmeldenummer: 98919137.4
(22) Anmeldetag: 01.04.1998
(51) Int. Cl.: C12N 15/70, C12Q 1/68, A61K 31/70

(54) **BAKTERIELLE PLASMIDE**
BACTERIAL PLASMIDS
PLASMIDES BACTERIENS

(30) Priorität: 02.04.1997 DE 19713543
(43) Veröffentlichungstag der Anmeldung: 02.02.2000
(73) Patentinhaber: PHARMA-ZENTRALE GMBH, 58313 Herdecke (DE)
(72) Erfinder: HACKER, Jörg, D-97218 Gerbrunn (DE); SONNENBORN, Ulrich, D-44799 Bochum (DE); SCHULZE, Jürgen, D-14558 Bergholz-Rehbrücke (DE); BLUM-OEHLER, Gabrielle, D-97072 Würzburg (DE); MALINKA, Jürgen, D-59379 Selm (DE); PROPPERT, Hans, D-58095 Hagen (DE)
(74) Vertreter: Schaeffer, Michael
(86) Internationale Anmeldenummer: PCT/EP1998/001720
(87) Internationale Veröffentlichungsnummer: WO 1998/044134

(56) Entgegenhaltungen:
- WO-A-96/34107
- CANNON, P. M. ET AL.: "Complete nucleotide sequence and gene organization of plasmid NTP16." PLASMID, Bd. 27, Nr. 3, Mai 1992, Seiten 220-30, XP002085747
- STIEGLITZ, H. ET AL.: "Identification of a 2-Md plasmid from Shigella flexneri associated with reactive arthritis." ARTHRITIS AND RHEUMATISM, Bd. 32, Nr. 8, August 1989, Seiten 937-946, XP002085748
- STIRLING, C.J. ET AL.: "The arginine repressor is essential for plasmid-stabilizing site-specific recombination at the ColE1 cer locus." EMBO JOURNAL, Bd. 7, Nr. 13, 1988, Seiten 4389-95, XP002085749 -& DATABASE EMBL - EMPRO Entry Pccg1, Acc.No. J01566; M33100, 15. Februar 1992 "Plasmid ColE1, complete genome." XP002085751
- YASUEDA, H. ET AL.: "Structural and functional organization of ColE2 and ColE3 replicons" MOLECULAR AND GENERAL GENETICS, Bd. 215, 1989, Seiten 209-16, XP002094006
- BOYD, A.C. ET AL.: "Characterization of the ColE1 mobilization region and its protein products" MOLECULAR AND GENERAL GENETICS, Bd. 217, 1989, Seiten 488-98, XP002094007
- BLUM, G. ET AL.: "Properties of Escherichia coli strains of serotype O6." INFECTION, Bd. 23, Nr. 4, Juli 1995, Seiten 234-6, XP002085750

## Beschreibung

Die Erfindung betrifft bakterielle Plasmide.

Plasmide sind kleine, extrachromosomale, meist zirkuläre und selbständig replizierende DNA-Moleküle, die in fast allen Bakterien und auch in einigen Eukaryonten sowie in den Mitochondrien vorkommen. Die Größe der Plasmide variiert zwischen etwa 1.5 bis 300 kb.

Bakterielle Plasmide sind in der Regel zirkulär. kovalent geschlossen und supercoiled. Häufig tragen sie Resistenzgene gegen Antibiotika oder Schwermetalle, Gene für die Metabolisierung untypischer Substrate oder Gene für eine Reihe von artspezifischen Charakteristika, wie metabolische Eigenschaften oder Virulenzfaktoren. Manche Plasmide können von einer Zelle in eine andere Zelle übertragen werden. Da die Pathogenität von Bakterien nach heutiger Ansicht zum Teil auch durch die Eigenschaften der Plasmide bedingt ist, besteht zunehmend ein größeres Interesse an der Aufklärung der Eigenschaften der Plasmid-DNA.

Von der Familie der Enterobacteriaceae, zu der 14 Hauptgattungen und 6 weitere Gattungen gehören, ist bekannt, daß diese unterschiedliche Eigenschaften ausbilden können. Typische Beispiele sind Escherichia, Salmonella und Klebsiella. Escherichia coli (E. coli) ist das klassische Objekt der Bakteriengenetik. Erst die Entdeckung und Charakterisierung verschiedener Virulenzfaktoren von E. coli ermöglichte es, allgemein zufriedenstellende Erklärungen dafür zu finden, daß Stämme dieser Art eine zum Teil extrem unterschiedliche Human- bzw. Tierpathogenität aufweisen, die von Avirulenz bis hochgradiger Virulenz, wie im Falle der sich neuerdings ausbreitenden als "EHEC" benannten Variante, reicht. So sind sowohl für extraintestinale als auch für intestinale E.-coli-Stämme bereits eine Reihe von Virulenzfaktoren beschrieben worden, die zum Teil gut charakterisiert sind. Für pathogene E.-coli-Stämme der Sero-Gruppe O6:K5 wurden Virulenzfaktoren wie z.B. Haemolysine und P-Fimbrienadhäsine gefunden, die nachweislich bei apathogenen Vertretern dieser Sero-Gruppe nicht auftreten.

Virulenzgene werden bei Enterobakterien in der Regel auf großen Plasmiden (ca. 60 kb) gefunden. Es gibt aber auch Enterobakterien mit kleinen, sogenannten kryptischen Plasmiden, deren Funktion bisher noch nicht sicher bestimmt werden konnte.

Da bekannt ist, daß im Falle von E. coli Virulenzfaktoren zumindest teilweise auch in den Genen der Plasmide vorhanden sind, besteht daher ein Bedarf nach weiteren Untersuchungen zur Auffindung und Charakterisierung von Plasmiden bei Enterobakterien und insbesondere Escherichia, um z.B. die Diagnose- und Therapiemöglichkeiten bei Erkrankungen nach Infektionen mit Enterobakterien zu verbessern. Die Plasmide bzw. ihre bakteriellen Träger oder entsprechende synthetisierte DNA können in der mikrobiologischen Analytik oder Diagnostik, medizinisch in der Therapie oder Prophylaxe und auch zu ernährungsphysiologischen oder probiotischen Zwecken Verwendung finden. Darüber hinaus sind Plasmide, und zwar insbesondere solche von E. coli, bekannte Expressionsvektoren in der Gentechnologie, so daß auch aus diesem Grunde ein Interesse an einer verbesserten Kenntnis der Eigenschaften derartiger Plasmide besteht.

Dazu wurden molekulargenetische Untersuchungen mit dem E.-coli-Stamm DSM 6601 durchgeführt. Die von diesem Stamm erhaltenen DNA-Sequenzen wurden mit Hilfe von Datenbankprogrammen einer DNA-Sequenzanalyse unterzogen und mit bereits vorliegenden DNA-Sequenzen anderer Bakterien verglichen.

Der Stamm DSM 6601 besitzt zwei kleine Plasmide mit einer Größe von 31 77 bzw. 5552 bp, die als pMUT1 bzw. pMUT2 bezeichnet wurden.

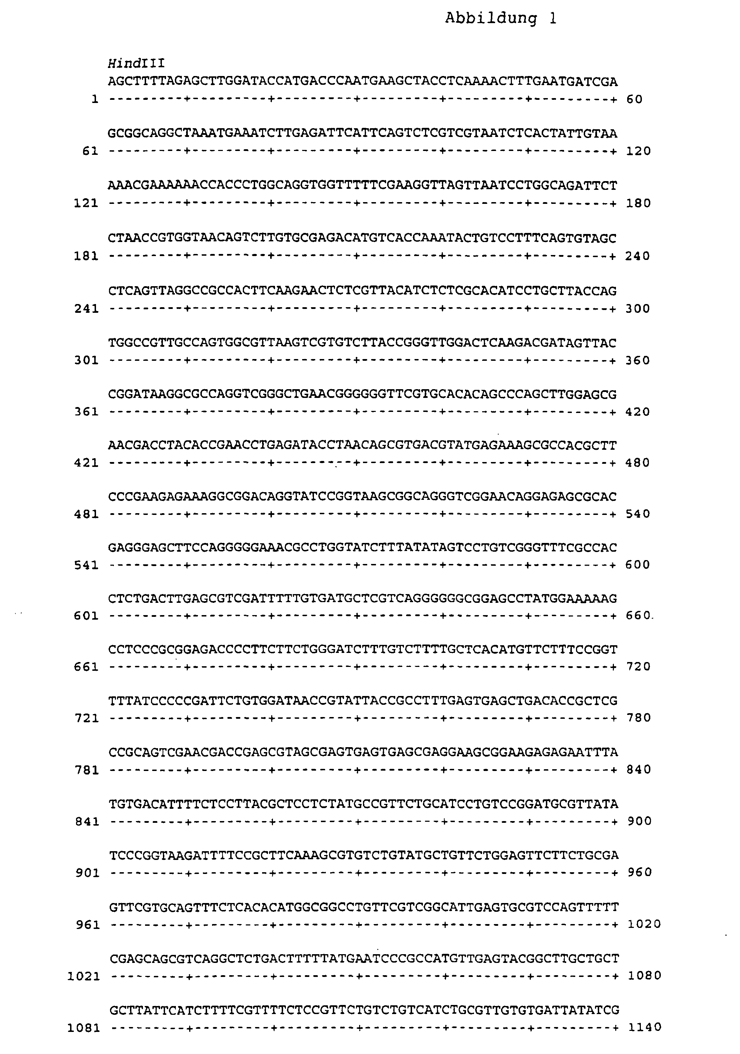

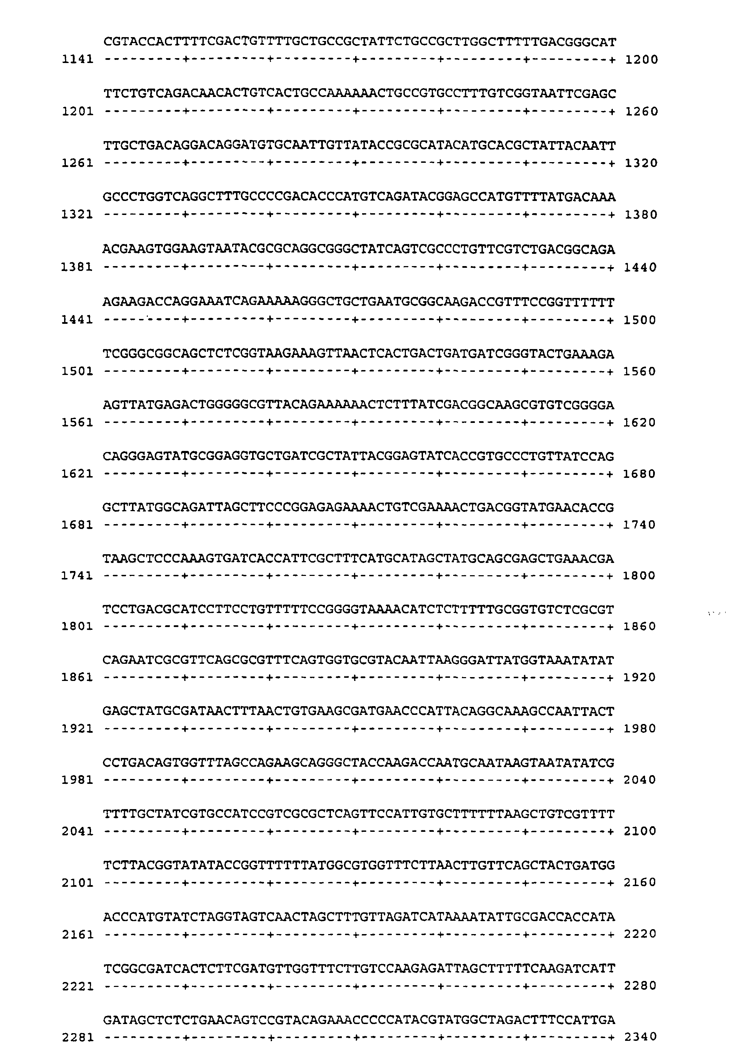

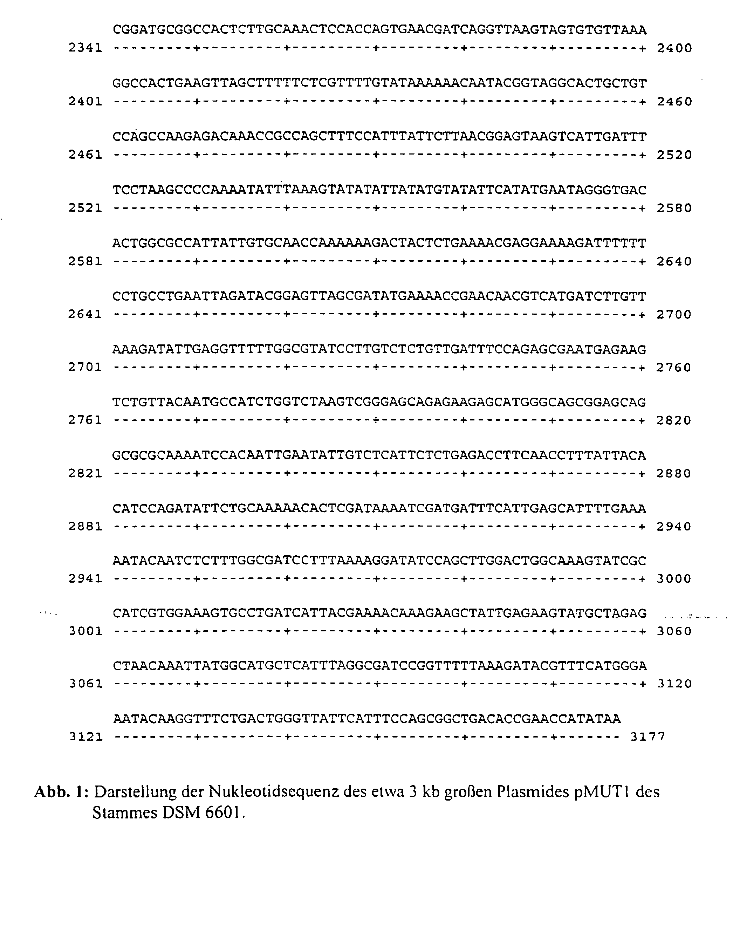

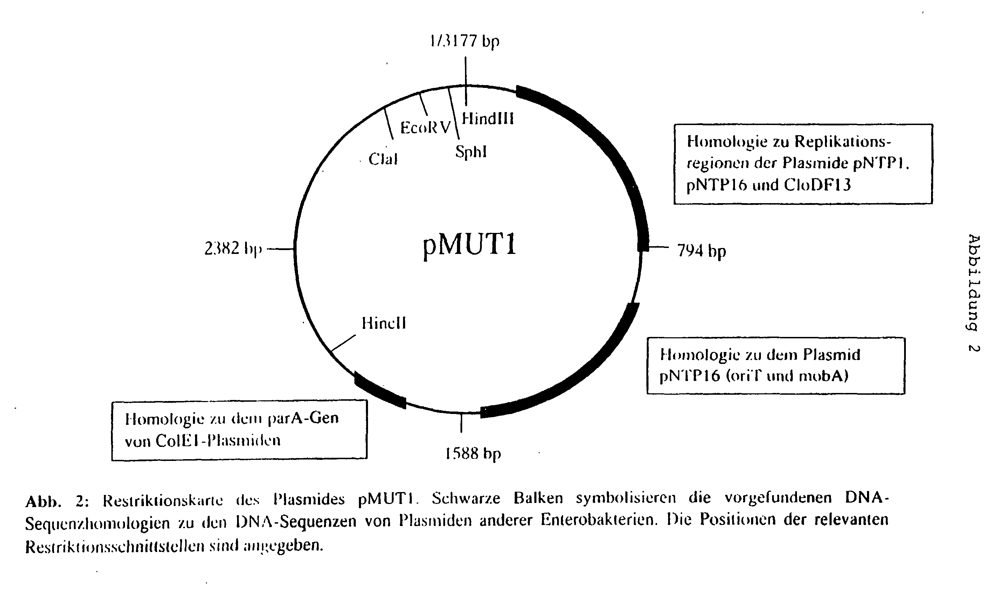

Die DNA des kleineren Plasmids pMUT1 wurde nach Restriktionsspaltung mit dem Enzym *Hind*III als lineares Fragment in den Vektor pUC18 subkloniert, anschließend wurde die DNA-Sequenz ermittelt. Diese ist aus der beigefügten Abbildung 1 ersichtlich. Die so erhaltene DNA-Sequenz wurde einem Homologievergleich mit der GenEMBL-Datenbank unterzogen; die Ergebnisse dieser Gegenüberstellung ergeben sich aus Abbildung 2.

Für diesen Vergleich wurde die *Hind*III-Schnittstelle als Position 1 festgelegt. Von der Position 200 bis 800 bp zeigt die DNA des Plasmides pMUT1 signifikante Homologien zu verschiedenen Reduplikationsstartstellen (origins of replication) anderer enterobakterieller Plasmide, speziell zu den Plasmiden NTP1, NTP16 und CloDF13. Im Bereich von Position 950 bp beginnt eine 570 bp große Homologie zu dem Plasmid NTP16, das ursprünglich aus Salmonella typhimurium isoliert wurde. Diese Homologie umfaßt das mobA-Gen, welches für die Mobilisierbarkeit des Plasmides NTP16 notwendig ist sowie einen origin of transfer (oriT). Darüber hinaus wurden von Position 1790 bis 1920 bp signifikante Homologien zu den Genen parA und cer gefunden, die für die Stabilität und die kontinuierliche Weitergabe und Verteilung von Plasmidmolekülen bei der Zellteilung von Bedeutung sind. Für die übrigen DNA-Regionen konnte keine signifikante Homologie identifiziert werden.

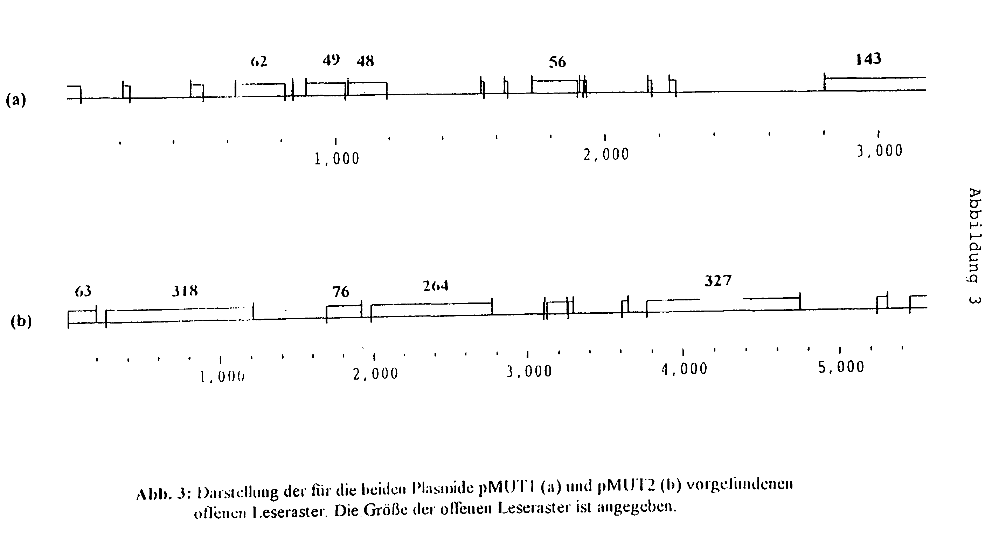

Darüber hinaus wurde die DNA-Sequenz des Plasmides pMUT1 in eine Aminosäure-Sequenz umgeschrieben und auf das Vorhandensein von offenen Leserastern analysiert. Es wurden 6 verschiedene Leserastermöglichkeiten untersucht. Es konnten insgesamt 5 offene Leseraster mit einer Größe von 143, 62, 56, 49 und 48 Aminosäuren gefunden werden. Eine grafische Darstellung der Analyse ist in Abbildung 3 gegeben.

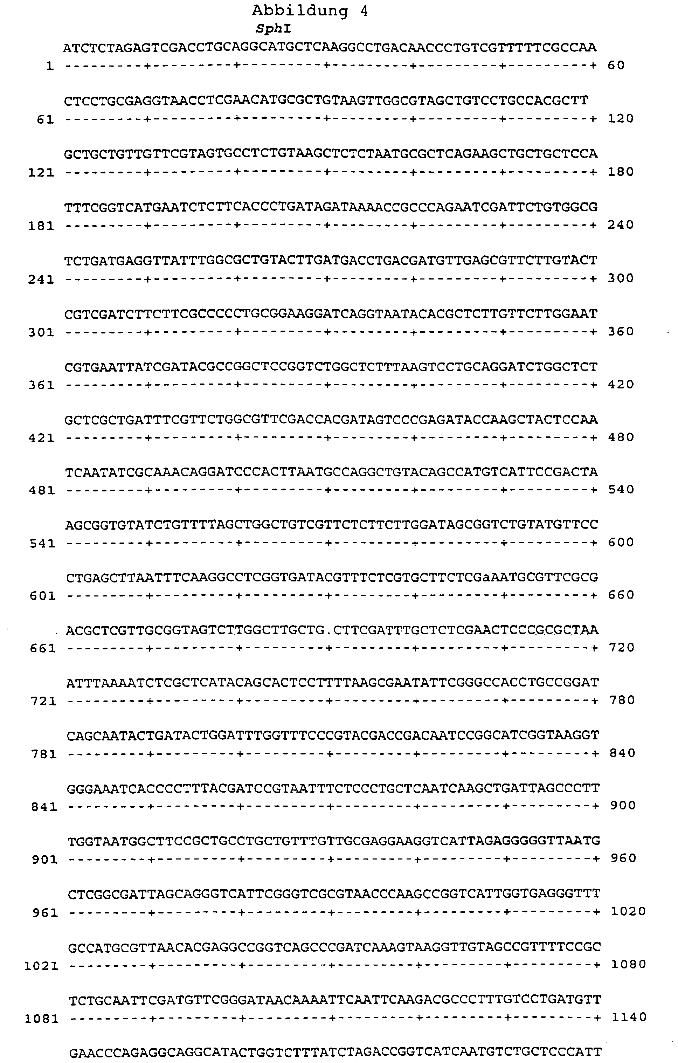

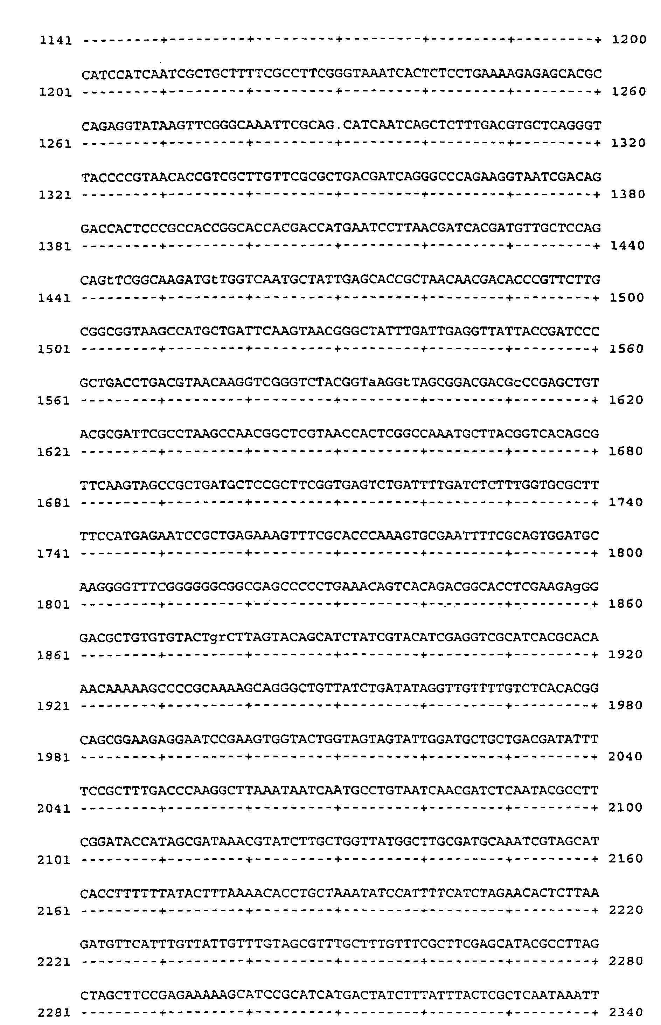

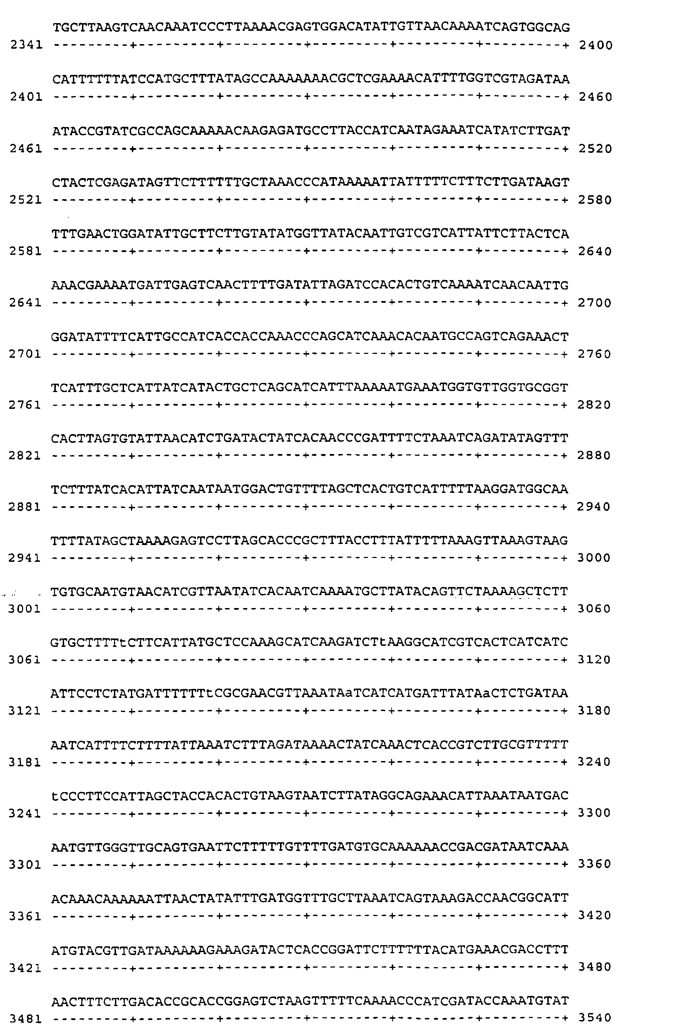

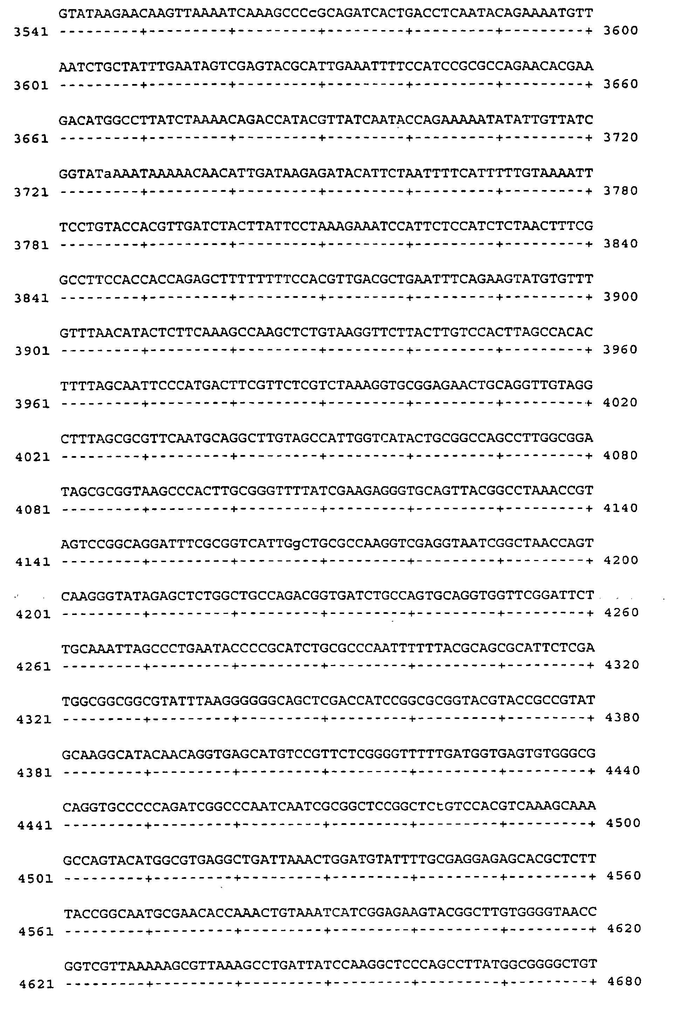

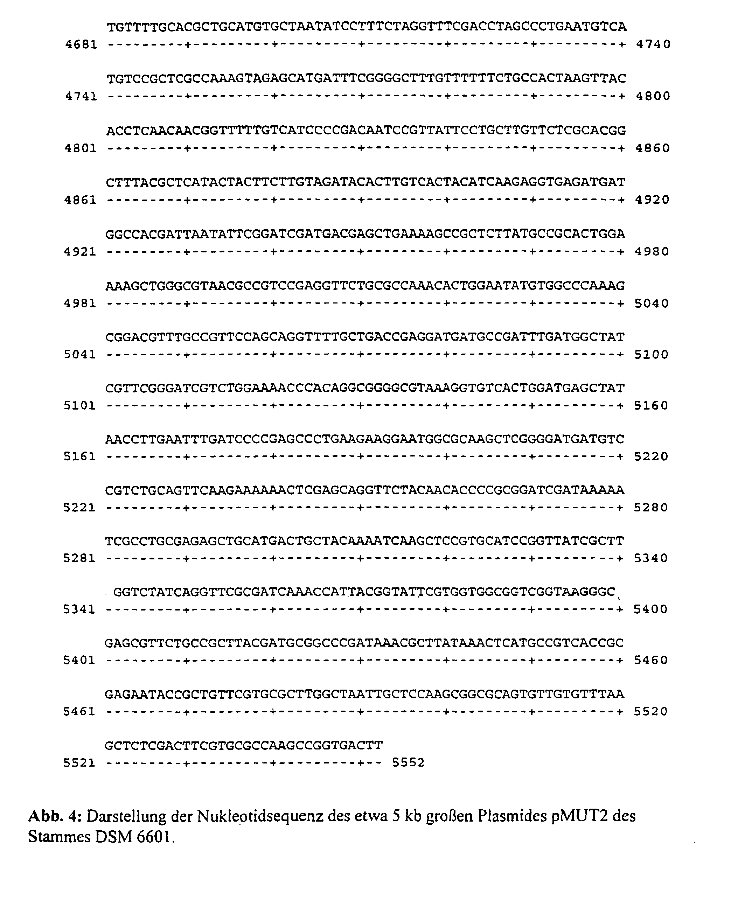

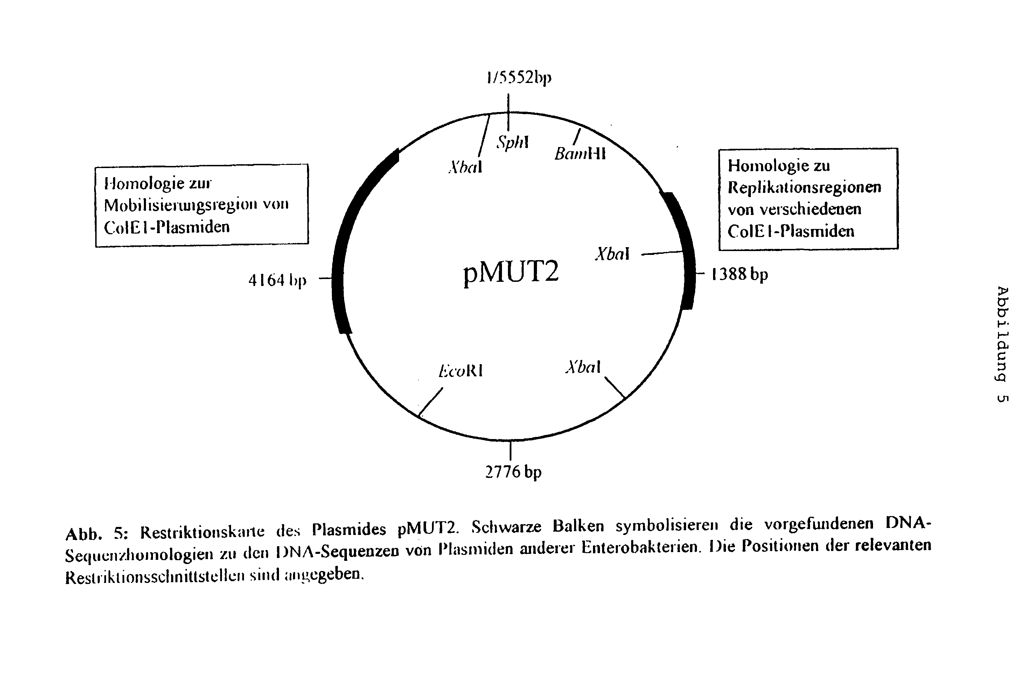

Die DNA des größeren Plasmides pMUT2 wurde nach Linearisierung mit dem Restriktionsenzym *Sph*I ebenfalls in den Vektor pUC18 subkloniert und anschließend vollständig sequenziert. Die DNA-Sequenz ergibt sich aus Abbildung 4. Die so erhaltene DNA-Sequenz wurde mit Hilfe des GenEMBL-Datenbankprogrammes auf Homologien zu bereits bekannten DNA-Sequenzen untersucht. Das Ergebnis ist in Abbildung 5 grafisch dargestellt. Die DNA des Plasmides pMUT2 zeigt signifikante Homologien zur replicon region verschiedener ColE1-Plasmide von E. coli in der Position 890 bis 1660 bp. Eine weitere signifikante Homologie zu ColE1-Plasmiden befindet sich in der Region von Position 3800 bis 4950 bp. Hierbei handelt es sich um Homologien zur Mobilisationsregion von ColE1-Plasmiden. Im Bereich von Position 3770 bis 4980 bp wurden Homologien zu einem Plasmid des Pasteurella-haemolytica-Stammes A1 gefunden. Auf diesem Plasmid befinden sich Gene, die bei Pasteurella für antimikrobielle Resistenzproteine kodieren. Die Homologie erstreckt sich jedoch über den intergenischen Bereich, so daß es sich möglicherweise auch um Sequenzen handelt, die für die Mobilisierbarkeit des Plasmides nötig sind.

Es wurden zwei Regionen identifiziert, die signifikante Homologien zu anderen enterobakteriellen Plasmiden aufweisen. Dabei handelt es sich um die origin-of-replication-Regionen und die mob-Regionen, die für die Mobilisierbarkeit der Plasmide notwendig sind. Für pMUT 2 konnte für die übrigen DNA-Abschnitte keine signifikante Homologie identifiziert werden.

Auch die DNA-Sequenz des Plasmides pMUT2 wurde anschließend in eine Aminosäure-Sequenz umgeschrieben und auf das Vorhandensein von offenen Leserastern analysiert. Das Ergebnis ist in Abbildung 3 ebenfalls grafisch dargestellt. Es wurden 5 offene Leseraster mit Aminosäure-Sequenzen in der Größenordnung von 327, 318, 264, 76 und 63 Aminosäuren gefunden.

Neben den bisher unbekannten Plasmiden pMUT 1 und pMUT 2 ist auch deren Kombination bisher in keinen E.-coli-Stämmen oder anderen Enterobakterien gefunden worden.

Das Vorkommen der Plasmide steht möglicherweise in Beziehung zu den metabolischen und medizinischen und/oder ernährungsphysiologisch bzw. probiotisch nutzbaren Eigenschaften des Stammes DSM 6601.

Die Untersuchung der Plasmide ermöglicht eine genauere Bestimmung und Analyse von Enterobakterien und insbesondere der Escherichia-Gruppe. Darüber hinaus bieten sich diese Plasmide als unbedenkliche Expressionsvektoren für die Gentechnologie an.

Im folgenden wird die Erfindung anhand der Beispiele näher erläutert:

### Beispiel 1:

### Plasmid-Isolierung

Die Isolierung der Plasmid-DNA erfolgte in Anlehnung an das Verfahren Birnboim et al. (Birnboim, A.C. und Doly, J. (1979) Nucl. Acids Res. 7:1513-1523 A rapid alkaline extraction procedure for screening recombinant plasmid DNA).

3 ml LB-Medium werden mit einer Bakterienkolonie beimpft und über Nacht bei 37° C geschüttelt. Diese Kultur wird in einem Eppendorfgefäß abzentrifugiert, der Medienrückstand wird mit einer Pipette entfernt. Das Zellsediment wird mit 100 µl Lösung I (50 mM Glukose; 10 mM EDTA, pH 8; 25 mM Tris-HCl, pH 8) resuspendiert. Nach 5 min Inkubation bei Raumtemperatur gibt man 200 µl Lösung II (0,2 N NaOH; 1 % SDS) dazu, mischt bis zum Aufklaren und läßt das Eppendorfgefäß weitere 5 min auf Eis stehen. Dann fügt man 150 µl Lösung III (3 M Na-Acetat, pH 4,8) hinzu, schüttelt kurz bis die chromosomale DNA flockig ausfällt und beläßt den Ansatz nochmals 5 min auf Eis. Die ausgefällte chromosomale DNA und die Zellreste werden 5 min in der Zentrifuge pelletiert und der Überstand mit der Plasmid-DNA wird in ein neues Gefäß überführt. Zur Reinigung der Plasmid-DNA werden 50 µl Phenol und 150 µl Chloroform/Isoamylalkohol (24:1) zugegeben und nach kurzem Schütteln 2 min abzentrifugiert. Die wäßrige Phase wird in ein neues Gefäß pipettiert. Die Plasmid-DNA wird mit 2 Volumina eiskaltem Ethanol ausgefällt und 10 min abzentrifugiert. Das Pellet wird mit 70 % Ethanol gewaschen und in der Speedvac getrocknet. Die Plasmid-DNA wird in 20 µl H₂O_{bidest} resuspendiert und bei -20°C aufbewahrt.

### Beispiel 2:

### DNA-Sequenzierung

Die DNA-Sequenzierung erfolgte in Anlehnung an das Verfahren von F. Sanger et al. (Sanger, F., Nicklen, S. und Coulson, A.R. (1977) Proc. Natl. Acad. Sci. USA 74: 5463-5467 DNA sequencing with chain terminating inhibitors)

Die DNA-Sequenzierung erfolgte mit dem T7-Sequenzier-Kit der Firma Phamacia LKB.

Für den Denaturierungsschritt werden 8 µl (1,5 bis 2 µg) Plasmid-DNA mit 2 µl 2 N NaOH gemischt; kurz abzentrifugiert und 10 min bei Raumtemperatur inkubiert. Die DNA wird mit 3 µl 3 M Na-Acetat, pH 4,8 sowie 7 µl H₂O_{bidest} und 60 µl eiskaltem Ethanol_{absolut} 15 min bei -70° C gefällt. Die gefällte DNA wird 10 min abzentrifugiert, mit 70 % Ethanol gewaschen und getrocknet.

Für die Annealing-Reaktion wird die denaturierte DNA in 10 µl H₂O_{bidest} suspendiert und mit 2 µl Annealing-Puffer und 2 µl Primer (40 ng) gemischt. Der Ansatz wird 20 min bei 37° C inkubiert, so daß eine Bindung des Primers an die Template-DNA erfolgen kann. Der Reaktionsansatz wird 10 min bei Raumtemperatur abgekühlt und dann entweder sofort für die Labelling-Reaktion verwendet oder bei -20° C eingefroren. Für die Labelling-Reaktion werden 3 µl Labelling-Mix, 1 µl [α-P³²]dATP und 2 µl T7-Polymerase (die T7-Polymerase wurde 1:5 mit Enzymverdünnungspuffer verdünnt) in den Annealing-Reaktionsansatz einpipettiert und nach kurzem Mischen 5 min bei Raumtemperatur inkubiert. Währenddessen werden die für die Terminationsreaktion bereits vorbereiteten Sequenziermixe (je 1 Gefäß mit 2,5 µl 'G'-, 'A'-, 'T'- und 'C'-Mix "short") bei 37° C vorgewärmt. Nach Ablauf der Labelling-Reaktion werden jeweils 4 µl davon zu den vier Sequenziermixen zugegeben und kurz mit der Pipette gemischt. Die Terminationsreaktionen werden 5 min bei 37° C inkubiert. Zum Beenden der Terminationsreaktionen werden je 5 µl Stop-Lösung zugegeben. Die Ansätze werden nun in einen Inkubator mit 95° C überführt, 2 min denaturiert und dann auf Eis gestellt. Je 2,5 µl der Reaktionen werden auf ein Sequenziergel [25,2 g Harnstoff, 22 ml H₂O_{bidest}, 6 ml 10x TBE, 10 ml Polyacrylamid (40 %), 2 ml Ammoniumpersulfat (16 mg/ml), 60 µl TEMED] in der Reihenfolge 'G', 'A', 'T', 'C' aufgetragen. Die Elektrophorese erfolgt bei 40 Watt und 1500 Volt für 4,5 h.

## Patentansprüche

1. Plasmid mit der in Abbildung 1 dargestellten DNA-Sequenz.

2. Plasmid mit der in Abbildung 4 dargestellten DNA-Sequenz.

3. DNA-Moleküle mit der in Abbildung 1 dargestellten Nucleotidfolge.

4. DNA-Moleküle mit der in Abbildung 4 dargestellten Nucleotidfolge.

5. Verwendung der Plasmide oder DNA-Moleküle nach Anspruch 1 bis 4 in der mikrobiologischen Analytik und/oder in vitro Diagnostik.

6. Verwendung der Plasmide oder DNA-Moleküle nach Anspruch 1 bis 4 als Expressionsvektoren.

## Claims

1. A plasmid with the DNA sequence illustrated in Fig. 1.

2. A plasmid with the DNA sequence illustrated in Fig. 4.

3. DNA molecules with the nucleotide sequence illustrated in Fig. 1.

4. DNA molecules with the nucleotide sequence illustrated in Fig. 4.

5. Use of the plasmids or DNA molecules according to Claims 1 to 4 in microbiological analysis and/or *in vitro* diagnostics.

6. Use of the plasmids or DNA molecules according to Claims 1 to 4 as expression vectors.

## Revendications

1. Plasmide comportant la séquence d'ADN représentée sur la figure 1.

2. Plasmide comportant la séquence d'ADN représentée sur la figure 4.

3. Molécule d'ADN présentant la séquence de nucléotides représentée sur la figure 1.

4. Molécule d'ADN présentant la séquence de nucléotides représentée sur la figure 4.

5. Utilisation d'un plasmide ou d'une molécule d'ADN, conforme à l'une des revendications 1 à 4, dans une analyse microbiologique et/ou dans un diagnostic *in vitro.*

6. Utilisation d'un plasmide ou d'une molécule d'ADN, conforme à l'une des revendications 1 à 4, en tant que vecteur d'expression.
